# FASCICULE DE BREVET EUROPEEN

(11) **EP 0 848 626 B1**
(45) Date de publication et mention de la délivrance du brevet: **24.10.2001**
(21) Numéro de dépôt: 96930201.7
(22) Date de dépôt: 04.09.1996
(51) Int. Cl.: A61M 25/00

(54) **CATHETER MULTICONDUITS, NOTAMMENT D'HEMODIALYSE**
KATHETER MIT MEHREREN LEITUNGEN, INSBESONDERE FÜR HAEMIDIALYSE
MULTILUMEN CATHETER, PARTICULARLY FOR HEMODIALYSIS

(30) Priorité: 05.09.1995 FR 9510573
(43) Date de publication de la demande: 24.06.1998
(73) Titulaire: Pourchez, Thierry, 62400 Bethune (FR)
(72) Inventeur: Pourchez, Thierry, 62400 Bethune (FR)
(74) Mandataire: Ecrepont, Robert
(86) Numéro de dépôt international: FR9601346
(87) Numéro de publication internationale: WO9709086

(56) Documents cités:
- EP-A- 0 453 234
- WO-A-93/16741
- WO-A-93/16752
- DE-U- 9 108 132
- US-A- 4 248 224
- US-A- 4 309 994

## Description

L'invention se rapporte à un dispositif dénommé cathéter multiconduits, utilisé pour la circulation d'au moins un fluide entre une cavité du corps d'un patient et un moyen de mise en circulation de ce fluide, tel un moyen de perfusion et/ou d'extraction de fluide.

L'invention intéresse plus particulièrement mais non exclusivement un cathéter d'hémodialyse.

Par cathéter multiconduits, on désigne un cathéter comprenant au moins deux conduits internes qui, définis par une paroi, sont chacun destinés au guidage d'au moins un fluide.

Un tel cathéter comprend également deux extrémités opposées dont l'une est dite distale, parce que notamment destinée à être placée dans une cavité du corps d'un patient pour y libérer et/ou y prélever le fluide par au moins canal, l'autre est dite proximale, parce que notamment destinée à être reliée à un moyen de mise en circulation de fluide, tel un moyen de perfusion et/ou d'extraction de fluide.

Avec les cathéters connus, le risque de dépôt de substances colmatantes sur les faces des canaux de perfusion et/ou d'extraction est notable. Un catheter selon le préambule de la revendication 1 est connu de EP-A-0 453 234.

Un résultat que l'invention vise à obtenir est un cathéter présentant un risque d'obstruction par colmatage qui est notablement réduit par rapport à celui des cathéters connus.

A cet effet, l'invention a pour objet un cathéter du type précité, ce cathéter étant caractérisé en ce que pour constituer son extrémité distale, il comprend au delà d'un point dit de division, situé à une distance déterminée de son extrémité proximale, au moins deux parties terminales allongées et distinctes qui :
- dans au moins une situation de repos du cathéter, s'étendent sensiblement parallèlement à l'axe longitudinal de ce cathéter, et ce, chacune sur une longueur déterminée, mesurable entre une extrémité libre et le point de division,
- sont chacune réalisées en matériau souple de manière à être flexibles au moins sous l'effet d'une action latérale due au déplacement d'un fluide,
- abritent au moins une fraction d'au moins un des conduits et présentent au moins un canal pour libérer et/ou prélever le fluide.

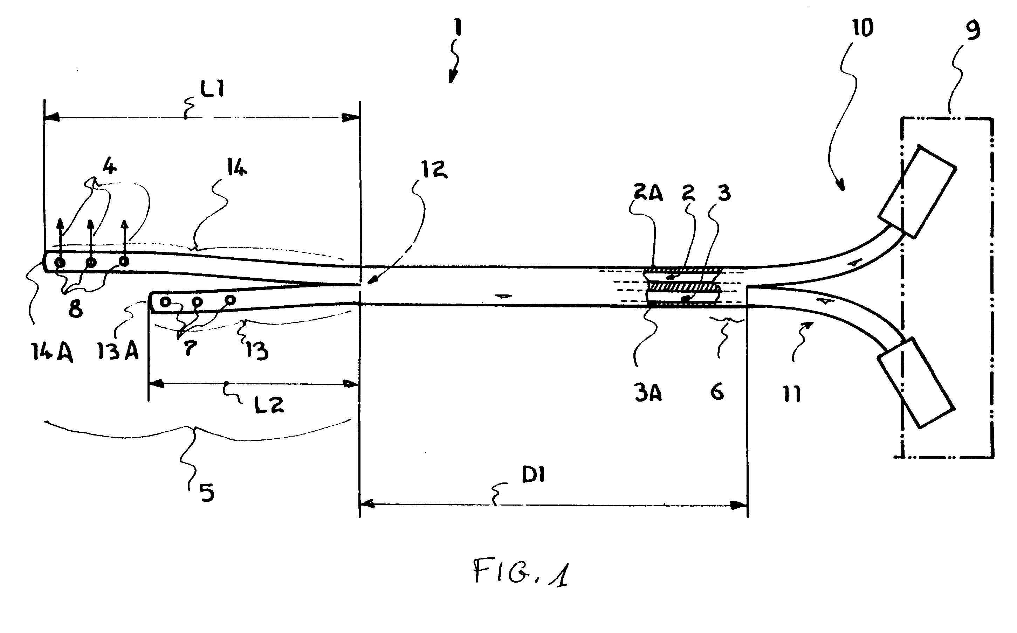

L'invention sera bien comprise à la lecture de la description ci-après faite à titre d'exemple non limitatif en regard du dessin ci-annexé qui représente une vue latérale d'un cathéter selon l'invention, dans une forme de réalisation.

En se reportant au dessin, on voit un dispositif allongé dit cathéter 1 comprenant :
- au moins deux conduits 2, 3 internes qui, définis par une paroi 2A, 3A, sont chacun destinés au guidage d'au moins un fluide 4,
- deux extrémités opposées 5, 6 dont l'une 5 est dite distale, parce que notamment destinée à être placée dans une cavité du corps d'un patient (non représenté) pour y libérer et/ou y prélever le fluide 4 par au moins canal 7, 8, l'autre 6 est dite proximale, parce que notamment destinée à être reliée à un moyen 9 de mise en circulation de fluide 4, tel un moyen de perfusion et/ou d'extraction de fluide.

Le fluide 4 a été représenté par des flèches 4.

Tel que cela est représenté, les canaux 7, 8 consistent en des perforations réalisées dans la paroi du cathéter qui définit chacun des conduits 2, 3.

Ce sont ces canaux qui permettent, soit la libération dans l'environnement du cathéter, d'un fluide contenu dans un conduit dudit cathéter, soit l'entrée dans un conduit de ce cathéter, d'un fluide environnant.

Par exemple, au moins l'un des conduits est destiné à la perfusion d'un fluide et au moins un autre conduit est quant à lui destiné à l'extraction de fluide.

Tel que cela est également représenté, les conduits 2, 3 de mise en circulation du fluide 4 consistent par exemple en des conduits juxtaposés, mais cela n'est pas limitatif pour l'invention.

Les dimensions et proportions du cathéter représenté ne sont pas limitatives pour l'invention.

A son extrémité proximale 6, le cathéter présente des organes 10, 11 pour le raccordement de chacun des conduits 2, 3 qu'il comprend au moyen 9 de mise en circulation de fluide 4.

L'invention n'ayant pas pour objet le moyen 9 de mise en circulation, ni les organes 10, 11 de raccordement à ce moyen 9, lesdits organes 10, 11 et le moyen 9 ne sont pas représentés en détail.

C'est au niveau de son extrémité distale 5 que le cathéter de l'invention est remarquable.

En effet, pour constituer son extrémité distale, le cathéter comprend au delà d'un point 12 dit de division, situé à une distance déterminée D1 de son extrémité proximale 6, au moins deux parties terminales 13, 14 allongées et distinctes qui :
- dans au moins une situation de repos du cathéter, s'étendent sensiblement parallèlement à l'axe longitudinal de ce cathéter, et ce, chacune sur une longueur déterminée L1, L2 mesurable entre une extrémité libre 13A, 14A et le point 12 de division,
- sont chacune réalisées en matériau souple de manière à être flexibles au moins sous l'effet d'une action latérale due au déplacement d'un fluide,
- abritent au moins une fraction d'au moins un des conduits 2, 3 et présentent au moins un canal 7, 8 pour libérer et/ou prélever le fluide.

Ces particularités techniques permettent que, lorsque l'extrémité distale du cathéter est placée dans l'axe d'un courant de fluide, tel un courant de fluide corporel, du fait de leur souplesse et de leur indépendance, les parties terminales, faseyent à la manière d'une voile orientée parallèlement au vent et, de ce fait, ne sont pratiquement pas sujettes à l'obstruction.

En effet, l'agitation et la flexion de ces parties terminales réduisent notablement le risque de dépôt de substances colmatantes sur les faces des canaux de perfusion et/ou d'extraction dont lesdites parties terminales sont pourvues.

Selon une autre caractéristique remarquable, d'une part, les parties terminales sont de longueurs L1, L2 différentes et, d'autre part, les canaux qui équipent ces parties terminales sont disposés de manière à déboucher chacun à un niveau différent du cathéter.

Conformément à une autre caractéristique de l'invention, les canaux qui équipent les parties terminales, d'une part, sont disposés en groupe sur chaque partie terminale et, d'autre part, ces groupes sont disposés à des niveaux différents du cathéter.

## Revendications

1. Cathéter (1) multiconduits de type comprenant :
- au moins deux conduits (2, 3) internes qui, définis par une paroi (2A, 3A), sont chacun destinés au guidage d'au moins un fluide (4),
- deux extrémités opposées (5, 6) dont l'une (5) est dite distale, parce que notamment destinée à être placée dans une cavité du corps d'un patient (non représenté) pour y libérer et/ou y prélever le fluide (4) par au moins canal (7, 8), l'autre (6) est dite proximale, parce que notamment destinée à être reliée à un moyen (9) de mise en circulation de fluide (4), tel un moyen de perfusion et/ou d'extraction de fluide,
ce cathéter étant **caractérisé en ce que**, pour constituer son extrémité distale, il comprend au delà d'un point (12) dit de division, situé à une distance déterminée D1 de son extrémité proximale (6), au moins deux parties terminales (13, 14) allongées et distinctes qui :
- dans au moins une situation de repos du cathéter, s'étendent sensiblement parallèlement à l'axe longitudinal de ce cathéter, et ce, chacune sur une longueur déterminée (L1, L2) mesurable entre une extrémité libre (13A, 14A) et le point (12) de division,
- sont chacune réalisées en matériau souple de manière à être flexibles au moins sous l'effet d'une action latérale due au déplacement d'un fluide, tel qu'un fluide corporel,
- abritent au moins une fraction d'au moins un des conduits (2, 3) et présentent au moins un canal (7, 8) pour libérer et/ou prélever le fluide.

2. Cathéter selon la revendication 1 **caractérisé en ce que**:
- d'une part, les parties terminales sont de longueurs (L1, L2) différentes et,
- d'autre part, les canaux qui équipent ces parties terminales sont disposés de manière à déboucher chacun à un niveau différent du cathéter.

3. Cathéter selon la revendication 2 **caractérisé en ce que** les canaux qui équipent les parties terminales, d'une part, sont disposés en groupe sur chaque partie terminale et, d'autre part, ces groupes sont disposés à des niveaux différents du cathéter.

## Patentansprüche

1. Katheter (1) mit Vielfachleitung der Art, umfassend:
- wenigstens zwei von einer Wand (2A, 3A) definierte interne Leitungen (2, 3) die jede zur Führung wenigstens einer Flüssigkeit (4) bestimmt ist,
- zwei gegenüberliegende äußere Ende (5, 6), von denen eines (5) ein sogenanntes distales ist, weil es insbesondere dazu bestimmt ist, in eine Körperhöhle eines Patienten (nicht dargestellt) zu werden, um dort die Flüssigkeit (4) durch wenigstens Kanal (7, 8) freizusetzen und / oder zu entnehmen, das andere (6) ein sogenanntes proximales ist, weil es insbesondere dazu bestimmt ist, mit einem Mittel (9) verbunden zu werden, das die Flüssigkeit (4) in Umlauf bringt, wie zum Beispiel ein Mittel zur Flüssigkeits-Perfusion und / oder -Extraktion,
wobei dieser Katheter **dadurch gekennzeichnet ist, daß** er, um sein distales äußeres Ende zu bilden, oberhalb eines sogenannten Teilungspunktes (12), der sich in einer bestimmten Entfernung D1 seines proximalen äußeren Endes (6) befindet, wenigstens zwei längliche und verschiedene Endteile (13, 14) hat, die:
- sich in wenigstens einer Ruhesituation des Katheters deutlich parallel zur Längsachse dieses Katheters erstrecken, und zwar jedes auf eine bestimmte meßbare Länge (L1, L2) zwischen einem freien äußeren Ende (13A, 14A) und dem Teilungspunkt (12),
- jedes aus biegsamen Material realisiert sind, daß sie wenigstens unter der Wirkung einer lateralen Einwirkung aufgrund der Verschiebung einer Flüssigkeit, wie zum Beispiel einer Körperflüssigkeit, flexibel sind,
- wenigstens einen Bruchteil wenigstens einer der Leitungen (2, 3) aufnehmen und wenigstens einen Kanal (7, 8) aufweisen, um die Flüssigkeit freizusetzen und / oder zu entnehmen.

2. Katheter gemäß Anspruch 1, **dadurch gekennzeichnet, daß**:
- einerseits die Endteile von verschiedener Länge (L1, L2) sind, und
- andererseits die Kanäle, die diese Endteile ausstatten, derart angeordnet sind, daß jeder in einer verschiedenen Höhe des Katheters einmündet.

3. Katheter gemäß Anspruch 2, **dadurch gekennzeichnet, daß** die Kanäle, die die Endteile ausstatten, einerseits auf jedem Endteil in Gruppen angeordnet sind und andererseits diese Gruppen auf verschiedenen Höhen des Katheters angeordnet sind.

## Claims

1. Multipipe catheter (1) of the type including :
- at least two internal pipes (2, 3) defined by a wall (2A, 3A) and each used to guide at least one fluid (4),
- two opposing extremities (5, 6) one (5) being called the distal extremity as it is the one to be placed in a cavity of the body of a patient (not shown) so as to free and/or remove the fluid (4) present there via at least one channel (7, 8), the other (6) being called the proximal extremity as this is the one intended to be connected to an element (9) for circulating this fluid (4), such as a fluid perfusion and/or extraction element,
this catheter being **characterised in that**, so as to constitute its distal extremity, it includes beyond a division point (12) situated at a specific distance D1 from its proximal extremity (6) at least two elongated and distinct terminal portions (13, 14) which :
- in at least one catheter idle situation extend approximately parallel to the longitudinal axis of said catheter over a specific length (L1, L2) able to be measured between one free extremity (13A, 14A) and the division point (12),
- are both made of a flexible material so as to be flexible at least under the effect of a lateral action due to movement of a fluid, such as a body fluid,
- protect at least one fraction of at least one of the pipes (2, 3) and have at least one channel (7, 8) for freeing and/or removing the fluid.

2. Catheter according to claim 1, **characterised in that**:
- firstly the terminal portions have different lengths (L1, L2), and,
- secondly the channels equipping these terminal portions are arranged so as to each open at a different level of the catheter.

3. Catheter according to claim 2, **characterised in that** the channels equipping the terminal portions are firstly arranged into groups on each terminal portion, and secondly these groups are placed at different levels of the catheter.
